# FASCICULE DE BREVET EUROPEEN

(11) **EP 1 617 814 B1**
(45) Date de publication et mention de la délivrance du brevet: **28.05.2008**
(21) Numéro de dépôt: 04742567.3
(22) Date de dépôt: 23.04.2004
(51) Int. Cl.: A61K 9/50

(54) **PROCEDE DE DISPERSION DE SUBSTANCES HYDROSOLUBLES OU HYDROPHILES DANS UN FLUIDE A PRESSION SUPERCRITIQUE**
VERFAHREN ZUR DISPERSION VON WASSERLÖSLICHEN ODER HYDROPHILEN SUBSTANZEN IN EINEM UNTER ÜBERKRITISCHEM DRUCK STEHENDEN FLUID
METHOD FOR THE DISPERSION OF WATER-SOLUBLE OR HYDROPHILIC SUBSTANCES IN A SUPERCRITICALLY PRESSURIZED FLUID

(30) Priorité: 25.04.2003 FR 0305108
(43) Date de publication de la demande: 25.01.2006
(73) Titulaire: ETHYPHARM, 78550 Houdan (FR); CENTRE NATIONAL DE LA RECHERCHE SCIENTIFIQUE (CNRS), 75016 Paris (FR)
(72) Inventeur: RICHARD, Joel, F-49400 Varrains (FR); DESCHAMPS, Frantz, F-49100 Angers (FR); LACROIX-DESMAZES, Patrick, F-34090 Montpellier (FR); BOUTEVIN, Bernard, F-34090 Montpellier (FR)
(74) Mandataire: Ahner, Francis
(86) Numéro de dépôt international: PCT/FR2004/000994
(87) Numéro de publication internationale: WO 2004/096173

(56) Documents cités:
- EP-A- 0 706 821
- WO-A-97/18234
- US-B1- 6 191 215
- DA ROCHA S R P, HARRISON K L, JOHNSTON KP: "Effect of Surfactants on the Interfacial Tension and Emulsion Formation between Water and Carbon Dioxide" LANGMUIR, vol. 15, 1999, pages 419-428, XP002276079
- DA ROCHA S R P ET AL: "Concentrated CO2-in-water emulsions with nonionic polymeric surfactants" JOURNAL OF COLLOID AND INTERFACE SCIENCE 01 JUL 2001 UNITED STATES, vol. 239, no. 1, 1 juillet 2001 (2001-07-01), pages 241-253, XP002276080 ISSN: 0021-9797
- JOHNSTON K P: "Block copolymers as stabilizers in supercritical fluids" CURRENT OPINION IN COLLOID AND INTERFACE SCIENCE 2000 UNITED KINGDOM, vol. 5, no. 5-6, 2000, pages 351-356, XP002276081 ISSN: 1359-0294
- DESIMONE J M ET AL: "DISPERSION POLYMERIZATIONS IN SUPERCRITICAL CARBON DIOXIDE" SCIENCE, AMERICAN ASSOCIATION FOR THE ADVANCEMENT OF SCIENCE,, US, vol. 265, 15 juillet 1994 (1994-07-15), pages 356-359, XP002060305 ISSN: 0036-8075
- LIM K T ET AL: "Synthesis and properties of semifluorinated block copolymers containing poly(ethylene oxide) and poly(fluorooctyl methacrylates) via atom transfer radical polymerisation" POLYMER, ELSEVIER SCIENCE PUBLISHERS B.V, GB, vol. 43, no. 25, 2002, pages 7043-7049, XP004389474 ISSN: 0032-3861
- HUSSAIN H ET AL: "SYNTHESIS AND CHARACTERIZATION OF POLY(ETHYLENE OXIDE) AND POLY(PERFLUOROHEXYLETHYL METHACRYLATE) CONTAINING TRIBLOCK COPOLYMERS" MACROMOLECULAR CHEMISTRY AND PHYSICS, WILEY VCH, WEINHEIM, DE, vol. 203, no. 14, 18 octobre 2002 (2002-10-18), pages 2103-2112, XP001190743 ISSN: 1022-1352

## Description

La présente invention concerne un procédé de dispersion et d'encapsulation de substances hydrosolubles ou hydrophiles dans un fluide supercritique.

De nombreux procédés industriels, notamment dans le domaine de la pharmacie, utilisent des dispersions de particules solides dans un solvant organique ou des émulsions d'un milieu aqueux dans un solvant organique. On citera notamment des procédés d'encapsulation par coacervation d'un agent de revêtement dissous dans un solvant organique provoquant le dépôt de cet agent de revêtement à la surface des particules en suspension dans la solution organique, ainsi que les procédés d'émulsion-extraction du solvant ou double émulsion.

Ces procédés font appel à de larges quantités de solvants organiques comme des solvants halogénés et sont accompagnés d'un coût environnemental important. Dans le domaine de la pharmacie, ces procédés nécessitent de mettre en place des méthodes de contrôle des solvants résiduels et éventuellement de réduction de la teneur en solvant résiduel des formulations pharmaceutiques. La tendance est à réduire ou même supprimer ces solvants dans les procédés destinés à la production de formulations pharmaceutiques. Il faut enfin ajouter que de nombreux principes actifs pharmaceutiques, comme par exemple les protéines thérapeutiques, peuvent être dénaturés par contact avec un solvant organique.

Le dioxyde de carbone (CO₂) à pression supercritique connaît des applications croissantes pour de nombreux procédés où il se substitue avantageusement aux solvants. Contrairement aux dispersions dans un solvant organique, les propriétés uniques du CO₂ à pression supercritique permettent, dans le cas de dispersions dans le CO₂ à pression supercritique, de procéder à une séparation des phases par simple décompression du milieu, conduisant ainsi à une récupération aisée des substances préalablement dispersées. Ces substances sont de plus exemptes de solvant organique résiduel. La formation de dispersions dans le CO₂ à pression supercritique doit donc offrir des possibilités avantageuses de substitution des solvants organiques dans des procédés industriels majeurs comme les séparations, les réactions ou les procédés de formation de matériaux spécifiques et notamment les procédés conduisant à des formulations pharmaceutiques sous forme de poudres.

Le demandeur s'est attaché à développer des procédés alternatifs sans solvant organique utilisant les propriétés avantageuses du CO₂ à pression supercritique. Le CO₂ à pression supercritique désigne ici du CO₂ porté à une pression voisine ou supérieure à sa pression critique (7,38 MPa). Il peut alors être soit à l'état liquide, soit à l'état supercritique. Ainsi, le brevet EP 0 784 506 B1 a pour objet un procédé de préparation de microparticules constituées de particules solides non solubles dans le CO₂ à pression supercritique enrobées par un agent de revêtement soluble dans le CO₂ à pression supercritique. L'étape clé de ce procédé est le dépôt de l'agent de revêtement sur les particules en suspension dans le fluide supercritique par modification contrôlée de la température et/ou de la pression du fluide supercritique. Les particules à enrober sont classiquement dispersées dans le fluide supercritique par agitation. Les améliorations apportées au procédé précité ont conduit le demandeur à rechercher des moyens d'améliorer les propriétés de dispersions de particules dans le fluide supercritique, avec l'objectif principal de stabiliser la dispersion et ainsi de diminuer les phénomènes d'agglomération des particules provoqués par une difficulté à disperser de manière homogène et stable des particules hydrophiles ou hydrosolubles dans le fluide à pression supercritique.

Les dispersions de particules solides sont réputées stables, si au cours du temps et sous l'effet des différentes interactions qui règnent dans le système dispersé, les particules restent individuelles, ne forment pas d'agrégats, réversibles ou irréversibles, et que la concentration en particules reste homogène en tout point du milieu dispersant, c'est-à-dire qu'aucune séparation de phase n'apparaît. Une perte de stabilité conduit le plus souvent d'abord à la formation d'agrégats de particules (floculation, coagulation) liée à l'apparition de forces attractives entre les particules, et ensuite à une séparation de phase (sédimentation ou crémage) due à la différence de densité entre les agrégats de grande taille ainsi formés et le milieu dispersant (effet de synérèse). Les mécanismes de stabilisation des dispersions de particules solides sont connus de l'homme de l'art et décrits dans les articles de référence de R.H. Ottewill (Journal of Colloid & Interface Science, Vol. 58, N° 2, pp 357-373, Février 1977) et de J.Th.G. Overbeek (Journal of Colloid & Interface Science, Vol. 58, N° 2, pp 408-422, Février 1977). Ils font intervenir les interactions électrostatiques, lorsque les particules sont porteuses de charges électriques à leur surface qui induisent des répulsions entre elles et empêchent la formation d'agrégats. Dans le cas de particules non porteuses de charges électriques, une stabilisation stérique des dispersions peut être obtenue en introduisant des chaînes polymères neutres (non ioniques), de longueur bien choisie, à la surface des particules. Ces chaînes peuvent être soit fixées chimiquement, soit adsorbées à la surface ou implantées physiquement dans la couche de surface des particules solides dispersées. Elles doivent également présenter une partie soluble en bon solvant dans la phase dispersante. Ces chaînes présentent ainsi un certain caractère tensioactif ou surfactant. La notion de stabilisation stérique a été introduite initialement par W. Heller et T.L. Pugh en 1954 (Journal of Chemical Physics, Vol. 22, p 1778, 1954). La stabilisation est générée d'une part par les forces de répulsion liées à l'enthalpie de mélange des chaînes stabilisantes en bon solvant, et d'autre part par l'élasticité de la couche de polymère formée à la surface des particules solides (origine entropique). Les contributions relatives de ces deux mécanismes dépendent principalement de la température, du caractère bon ou mauvais solvant de la phase dispersante, de la densité et de l'épaisseur de la couche de polymère stabilisante. Une revue complète des mécanismes de stabilisation stérique est donnée dans l'article de référence de D.H. Napper (Journal of Colloid & Interface Science, Vol. 58, N°2, pp390-407, 1977). Un exemple de dispersion de particules hydrophobes (polyacrylonitrile), stabilisées stériquement dans un solvant organique hydrophobe (toluène) par des chaînes de polystyrène adsorbées à leur surface, a été donné par A. Doroszwski et R. Lambourne (Journal of Colloid & Interface Science, Vol. 43, p 97, 1973). Plusieurs exemples de dispersions de particules solides hydrophiles (poly(12hydroxy-acide stéarique), poly(oxyéthylène)) dans des solvants organiques non aqueux (n-heptane, méthanol) ont été donnés par D.H. Napper (Journal of Colloid & Interface Science, Vol. 58, N°2, pp390-407, 1977). Des particules inorganiques, denses et hydrophiles, d'oxyde de fer ont aussi été dispersées et stabilisées stériquement dans la cyclohexanone par des chaînes de polyamide adsorbées à leur surface (T. Sato, Journal of Coatings Technology, Vol. 65, N° 825, pp113-121, 1993).

La plupart des surfactants commerciaux ne sont pas solubles dans le CO₂ à pression supercritique (Consani et al., Journal of Supercritical Fluids, 3 (1990) 51-65) et ne peuvent donc pas être utilisés pour stabiliser des dispersions dans le CO₂ à pression supercritique. De rares surfactants solubles dans le CO₂ à pression supercritique sont toutefois décrits dans la littérature. Il est bien connu que la majorité des polymères n'est pas soluble dans le CO₂ à pression supercritique à des températures compatibles avec des procédés destinés à la production de formulations pharmaceutiques (Kirby C.F. and McHugh, Chemical Review, 99 (1999) 565-602). Quelques familles de polymères CO₂-philes sont sensiblement solubles dans le CO₂ supercritique. Ces polymères sont majoritairement des polymères fluorés (Newman D.A. et al., Journal of Supercritical Fluids, 6 (1993) 205-210) comme les poly(fluoroéthers), les poly(fluoroacrylates), les poly(fluorométhacrylates), des silicones comme les poly(diméthyl siloxanes) (Hoefling et al., Journal of Supercritical Fluids, 6 (1993) 164-171), ou des poly(éther-carbonates) (Sarbu et al., Nature, 405 (2000) 165-167).

Des polymères CO₂-philes ont été mis en oeuvre pour synthétiser des surfactants utilisables dans le CO₂ à pression supercritique. Il s'agit soit de polymères CO₂-philes fonctionnalisés de manière à augmenter leur affinité pour les substances à disperser, soit de copolymères comportant des chaînons choisis parmi les polymères solubles dans le CO₂ (CO₂-solubles) préalablement décrits. Les applications principales de ces surfactants sont des procédés de polymérisation en dispersion dans le CO₂ supercritique où ces surfactants permettent une stabilisation stérique des particules de polymères en cours de formation. Les chaînons destinés à interagir avec les particules de polymère en formation sont des chaînons hydrophobes comme par exemple des polystyrènes, des poly(vinyl acétates) ou des poly(méthyl méthacrylates).

Ces composés sont tous des surfactants du type CO₂-phile/hydrophobe. Ils ne sont donc pas utilisables pour stabiliser des dispersions de substances hydrophiles liquides ou solides dans le CO₂ à pression supercritique, car ils sont dépourvus d'une partie hydrophile pouvant interagir avec des matériaux hydrophiles et se fixer à leur surface.

Des surfactants CO₂-philes à tête ionisable ont été synthétisés et évalués pour la formation d'émulsions d'eau dans le CO₂ supercritique. Ces composés sont constitués d'une partie CO₂-phile, comme des polymères fluorés ou des poly(siloxanes) et d'une courte tête ionisée pouvant interagir électrostatiquement avec la phase aqueuse à disperser. Ces surfactants ont, dans certains cas particuliers, permis la formation d'émulsions ou de microémulsions d'eau dans le CO₂ supercritique (Hoefling T.A. et al., Journal of Physical Chemistry, 95 (1991) 7127-7129). Ils ne sont toutefois pas utilisables pour stabiliser des dispersions de particules solides hydrophiles dans le CO₂ supercritique, c'est à dire dans un milieu non aqueux. La stabilisation de telles dispersions nécessite en effet une interaction forte du surfactant avec la surface des particules à disperser permettant la fixation permanente des surfactants polymères à leur surface. Elle est hors de portée des surfactants portant une courte tête polaire ionisable, qui est de plus non ionisée dans un milieu non aqueux.

Des tentatives de stabilisation stérique de substances inorganiques comme des particules de silice hydrophile ont été publiées (L. Calvo et al., Journal of Supercritical Fluids, 16 (2000) 247-260). Il est démontré dans cette étude que l'utilisation de surfactants solubles dans le CO₂ supercritique, comme un perfluoropolyéther portant une courte tête ionique carboxylate d'ammonium, ou un copolymère polystyrène-poly(1,1-dihydro-perfluorooctylacrylate), ou encore un copolymère poly(méthyl méthacrylate-co-hydroxyéthyl méthacrylate) greffé par des polymères polyfluoropolyéther, ne permet pas la stabilisation stérique de particules de silice hydrophile d'une taille supérieure à 1,2 µm dans le CO₂ supercritique à partir d'une dispersion de particules polydispersées (distribution granulométrique large) de silice d'une taille moyenne de 3µm dont 90% ont une taille supérieure à 1,5 µm. Si une suspension des particules de plus faible diamètre, compris-entre 0,7 et 1,2 µm, est stabilisée sur une courte durée, seule une infime fraction, inférieure à 2,5%, de la masse de particules de silice à disperser peut être stabilisée par ces surfactants. Ces surfactants, pourtant solubles dans le CO₂ supercritique et capables de stabiliser des dispersions de substances hydrophobes dans le CO₂ supercritique, ne permettent donc pas de stabiliser de manière efficace des dispersions de particules de silice hydrophile dans le CO₂ supercritique.

L'ensemble des surfactants CO₂-phile actuellement connu ne permet donc pas de préparer des dispersions stables de substances solides hydrosolubles ou hydrophiles dans le CO₂ à pression supercritique.

Les inventeurs ont trouvé de manière surprenante que l'utilisation de copolymères à blocs composés d'au moins un bloc CO₂-phile et d'au moins un bloc hydrophile non ionique, de composition et de masse molaire adaptées, permettent de préparer des dispersions stables de substances hydrophiles dans le CO₂ à pression supercritique.

Les copolymères sont notamment choisis dans le groupe constitué par des copolymères diblocs ou des copolymères triblocs, de préférence des copolymères diblocs.

Les copolymères triblocs peuvent répondre, soit à la formule (1)

hydrophile/CO₂-phile/hydrophile (1),

soit à la formule (2)

CO₂-phile/hydrophile/CO₂-phile (2),

dans lesquelles respectivement les groupes hydrophiles ou CO₂-philes peuvent être identiques ou différents.

La présente invention concerne l'utilisation de surfactants spécifiquement conçus pour préparer des dispersions stables de substances hydrosolubles ou hydrophiles dans un fluide à pression supercritique. Ces substances sont à l'état solide ou liquide dans les conditions de température et de pression du procédé et sont constituées de matières majoritairement hydrosolubles ou hydrophiles et non solubles dans le CO₂ à pression supercritique.

Le demandeur a conçu et synthétisé de nouveaux copolymères à blocs comprenant au moins un bloc CO₂-phile et au moins un bloc hydrophile non ionique. Ces copolymères se sont révélés aptes à stabiliser des dispersions de particules solides hydrophiles dans le CO₂ à pression supercritique. Ils peuvent être notamment employés pour améliorer le procédé d'encapsulation décrit dans le brevet EP 0 784 506 B1. Les copolymères blocs sont utilisés à la fois comme agents de stabilisation de la dispersion de particules hydrophiles et/ou comme agent de revêtement. Ces copolymères peuvent aussi être employés comme surfactants pour stabiliser une dispersion de particules à enrober, en conjonction avec des agents de revêtement classiquement utilisés dans le procédé précité comme des lipides, des cires, des polymères ou tout composé suffisamment soluble dans le CO₂ à pression supercritique et utilisable dans la mise en oeuvre du procédé précité.

Il a été découvert dans le cadre de la présente invention que ces surfactants particuliers peuvent aussi être utilisés pour la formation et la stabilisation stérique d'émulsions d'eau ou de solutions aqueuses dans le CO₂ à pression supercritique.

Le procédé objet de l'invention est défini dans les revendications. Il est particulièrement adapté à la production de formulations pharmaceutiques dont les procédés d'obtention comprennent une étape de dispersion de substances hydrosolubles ou hydrophiles dans un milieu dispersant constitué de CO₂ à pression supercritique.

Le milieu dispersant est du CO₂ porté à une pression voisine ou supérieure à la pression critique (7,38 MPa), de préférence entre 5 MPa et une pression de 70 MPa, encore plus préférentiellement entre la pression critique et une pression de 30 MPa. La température est comprise entre 0°C et 100 °C, et de préférence entre 15°C et 60°C.

Le milieu dispersant peut éventuellement contenir un entraîneur. Au sens de la présente invention, un entraîneur est défini comme une substance volontairement ajoutée au fluide supercritique en petites quantités, de manière à augmenter la solubilité d'une ou plusieurs substance(s) dans le dit fluide supercritique. Quand un tel entraîneur est présent en petite quantité dans le fluide supercritique (environ <5 %), cette quantité est si faible qu'elle est essentiellement sans effet sur les conditions nécessaires à l'entrée dans l'état supercritique du composant principal du fluide supercritique, mais augmente beaucoup la solubilité du matériau polymère. A titre d'exemple, on peut utiliser comme entraîneur, les cétones, les alcools, les esters et les solvants chlorés ou d'autres solvants organiques et les plastifiants. De tels entraîneurs sont particulièrement utilisés lorsque le polymère est peu soluble dans le CO₂ à pression supercritique.

Dans le cas de dispersions de substances solides dans les conditions du procédé, les particules à disperser sont constituées d'un matériau hydrophile ou hydrosoluble, non soluble dans le fluide à pression supercritique, notamment des particules contenant un principe actif.

Des principes actifs hydrophiles ou des principes actifs solubilisés ou dispersés dans une matrice hydrosoluble ou hydrophile sont particulièrement employés dans la formation de dispersions par le procédé de l'invention.

Les principes actifs sont choisis dans le groupe comprenant (i) les produits pharmaceutiques, notamment les analgésiques, les antipyrétiques, l'aspirine et ses dérivés, les antibiotiques, les anti-inffammatoires, les antiulcéreux, les antihypertenseurs, les neuroleptiques, les antidépresseurs, les oligonucléotides présentant une activité thérapeutique, les peptides présentant une activité thérapeutique et les protéines présentant une activité thérapeutique, (ii) les produits cosmétiques, notamment les autobronzants et les anti-UV, (iii) les produits alimentaires comme par exemple les vitamines.

Les protéines ou peptides thérapeutiques utilisés dans ces formulations sont choisis préférentiellement parmi la protéine correspondant à l'hormone parathyroïde (PTH), l'hormone de croissance (GH), les interférons α, β, ou γ, l'érythropoiétine α ou β (EPO), le facteur stimulateur de colonies de granulocytes (GCSF), le facteur stimulateur de colonies macrophages de granulocytes (GMSCF), le peptide vasoactif intestinal (VIP), l'hormone libératrice de thyrotopine (CRH), l'arginine vasopressine (AVP), l'angiotensine, l'insuline, la somatotropine, l'activateur de tissu plasminogène, les facteurs de coagulations VIII et IX, la glucosylcéramidase, la lénograstine, la molgramostine, la filgrastine, les interleukines, la dornase α, , la PEG-L-asparaginase, la PEG-adenosine deaminase, l'hirudine, l'eptacog α, les facteurs de croissances nerveuses, l'hormone libératrice d'hormone lutéinisante (LHRH), ses dérivés et ses analogues, la somatostatine et ses dérivés, la triptoréline, la bombésine, la calcitonine, le peptide libérateur de gastride, le facteur libérateur d'hormone de croissance et l'amyline.

Les particules contenant un principe actif issues d'un procédé de précipitation ou de cristallisation à l'aide de solvants organiques ou de fluides à pression supercritique, de lyophilisation, de nébulisation, de séchage, de broyage, susceptibles d'être dispersées dans le fluide à pression supercritique, sont préférentiellement constituées d'une matrice solide contenant le principe actif, notamment une protéine ou un peptide thérapeutique et éventuellement des excipients comme des agents de charge, des stabilisants de la protéine, des cryoprotecteurs et des lyoprotecteurs. Parmi les excipients, on peut citer de façon non exhaustive, les sels de phosphates et autres composés ioniques, les sucres, les polyols, des protéines de charge comme des albumines, des surfactants, et toutes autres substances utilisées pour stabiliser des formulations solides de protéines thérapeutiques existantes ou à venir. Ces particules contenant des principes actifs, notamment des protéines ou peptides sont pour la plupart hydrophiles et difficiles à disperser dans le CO₂ à pression supercritique.

La taille des particules dispersées par le procédé objet de l'invention est comprise entre 0,05 µm et 800 µm, et de préférence entre 0,1µm et 100 µm.

Les copolymères utilisés dans le procédé de l'invention sont des copolymères à blocs composés d'un bloc CO₂-phile et d'un bloc hydrophile non ionique. La nature des deux blocs, et leur rapport molaire, sont ajustables et permettent une modulation fine des propriétés de stabilisation de dispersions de solides ou de liquides hydrophiles non solubles dans le CO₂ à pression supercritique.

Le bloc CO₂-phile des copolymères à blocs est choisi avantageusement parmi les polymères solubles dans le CO₂ à pression supercritique.

Les copolymères à blocs sont avantageusement des copolymères solubles dans le CO₂ à pression supercritique. La solubilité minimale des copolymères à blocs selon l'invention est de 0,05% m/m, et de préférence de 0,2% m/m, à au moins une température définie et comprise entre 0°C et 100°C, de préférence définie et comprise entre 15°C et 60°C, et à au moins une pression définie et supérieure à la pression critique du CO₂, de préférence inférieure à 70 MPa et encore plus préférentiellement inférieure à 30 MPa.

La masse molaire des copolymères à blocs objets de l'invention et le rapport molaire des deux blocs CO₂-philes et hydrophiles doivent être choisis de manière à donner au copolymère une solubilité suffisante dans le CO₂ à pression supercritique et avantageuse pour le procédé objet de l'invention, dans des conditions de température et de pression avantageuses techniquement et économiquement et permettant la mise en oeuvre de substances fragiles comme les principes actifs pharmaceutiques.

La masse molaire et le rapport molaire sont également choisis pour obtenir les propriétés de stabilisation de dispersions désirées. Sur ces considérations de solubilité et de stabilisation de dispersions, il a été trouvé dans le cadre de la présente invention que la masse molaire moyenne en nombre (Mn) du copolymère doit être choisie entre 1 000 et 200 000 g/mol et plus particulièrement entre 4 000 et 50 000 g/mol. La masse molaire moyenne en nombre du bloc hydrophile est comprise entre 500 et 20 000 g/mol et préférentiellement entre 1 000 et 10 000 g/mol. Le rapport massique du bloc CO₂-phile sur le bloc hydrophile, défini comme le rapport des masses molaires moyennes en nombre, est compris entre 1 et 50, et préférentiellement entre 1 et 20.

Parmi les polymère solubles dans le CO₂ utilisés dans la synthèse des copolymères à blocs, on peut citer en particulier les polymères fluorés et les poly(siloxanes). Parmi les polymères fluorés, les polymères utilisés sont avantageusement choisis parmi les poly(fluoroéthers), les poly(fluoroalkyl méthacrylates), les poly(fluoroalkyl acrylates), et particulièrement les poly(1,1-dihydro-perfluorooctyl acrylates) ou PFOA et les poly(1,1,2,2-tétrahydro-perfluorodécyl acrylates) ou PFDA.

Le bloc hydrophile non ionique est choisi avantageusement parmi les polymères hydrophiles biocompatibles, notamment dans le groupe comprenant les polysaccharides, les polymères cellulosiques hydrophiles, l'alcool polyvinylique, les polyols, les homo- et copolymères d'oxyde d'éthylène. De préférence le bloc hydrophile est un poly(oxyde d'éthylène) ou POE.

Les copolymères à blocs utilisés selon le procédé de l'invention sont avantageusement des copolymères à blocs composés d'un bloc poly(1,1,2,2-tétrahydro-perfluorodécyl acrylate) et d'un bloc poly(oxyde d'éthylène) **ou sont** des copolymères à blocs du type POE-b-PFDA.

Parmi les copolymères triblocs selon l'invention, on peut citer de façon non limitative les copolymères triblocs PFDA-b-POE-PFDA et les copolymères triblocs POE-b-PFDA-b-POE.

La synthèse de ces copolymères est effectuée par toute voie synthétique adaptée et offrant l'accès à la composition désirée du copolymère en terme de nature des blocs CO2-philes et hydrophiles, de masse molaire et de rapport molaire des deux blocs. La synthèse s'effectue de préférence par un procédé de télomérisation radicalaire conventionnelle.

Les copolymères peuvent être purifiés après synthèse par l'ensemble des procédés couramment utilisés à cet effet comme les précipitations, les purifications par chromatographie, et de préférence par extraction ou fractionnement à l'aide de CO₂ à pression supercritique en raison de leur solubilité intrinsèque dans ce milieu. Cette extraction utilise les dispositifs classiques d'extraction et fractionnement par fluide supercritique discontinus ou continus.

La dispersion de substances hydrophiles est effectuée par tout moyen couramment utilisé pour provoquer la dispersion de substances solides ou liquides dans un fluide. Dans le cas de dispersions de particules solides, les moyens de dispersion préférés pour la présente invention sont l'agitation par usage d'un mobile en rotation et la fluidisation par usage d'un lit fluidisé par du CO₂ à pression supercritique.

L'usage de ces copolymères à blocs est particulièrement bénéfique dans les procédés de revêtements ou de modification de substances dispersées dans le CO₂ à pression supercritique. Ces procédés peuvent en effet être améliorés par une stabilisation de la dispersion, limitant les phénomènes d'agrégation.

L'invention est donc particulièrement utile dans les procédés d'encapsulation de particules d'intérêt pharmaceutique, destinés par exemple à améliorer le comportement rhéologique des poudres, à améliorer la stabilité de la formulation, à modifier leurs propriétés de surface, ou à leur conférer des propriétés de libération contrôlée.

La présente invention est particulièrement adaptée aux procédés de revêtement de particules dispersées dans le CO₂ à pression supercritique, comme le procédé décrit dans le brevet EP 0 784 506 B1 où une modification contrôlée de la température et/ou de la pression du CO₂ à pression supercritique permet le dépôt contrôlé d'un agent de revêtement soluble dans le CO₂ à pression supercritique. Les copolymères à blocs sont alors utilisés principalement en tant que surfactants permettant la stabilisation de la dispersion et éventuellement comme agents de revêtement, seuls ou en mélange avec d'autres agents de revêtement.

Les exemples et les figures 1 à 6 et 8 à 10 qui suivent illustrent l'invention.

Les exemples 7 et 11 sont des exemples comparatifs.

La figure 1 représente l'aspect des particules en microscopie électronique à balayage qui ont été soumises à un procédé d'encapsulation tel que décrit dans l'exemple 5.

La figure 2 représente une photographie d'une émulsion d'une solution aqueuse d'amarante dans le CO₂ supercritique à une température de 40°C et une pression de 30 MPa, obtenue en présence de copolymères à bloc POE-b-PFDA selon le procédé de l'invention.

La figure 3 représente une photographie d'un mélange d'une solution aqueuse d'amarante et de CO₂ supercritique à une température de 40°C et une pression de 30 MPa, sous agitation mais en l'absence de copolymère à bloc POE-b-PFDA.

La figure 4 représente une photographie d'une émulsion d'une solution aqueuse d'amarante dans le CO₂ liquide à une température de 20°C et une pression de 30 MPa, obtenue selon le procédé de l'invention.

### Exemple 1: Synthèse d'un copolymère à bloc POE-b-PFDA

Cet exemple décrit la synthèse d'un copolymère à blocs composé d'un bloc poly(oxyde d'éthylène) de masse molaire moyenne en nombre (Mn) 2 000 g/mol (POE 2000) et d'un bloc poly(1,1,2,2-tétrahydro-perfluorodécyl acrylate) (PFDA), soit un copolymère dibloc du type POE-b-PFDA. La synthèse est effectuée par télomérisation radicalaire conventionnelle.

Dans une première étape, on synthétise un télogène macromoléculaire (I) à partir du POE 2000.

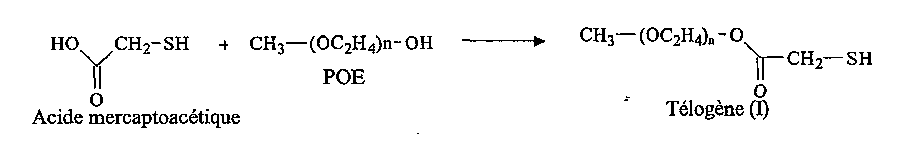

Dans un deuxième temps, on effectue la télomérisation de l'acrylate fluoré 1,1,2,2-tétrahydro-perfluorodécyl acrylate (FDA) en présence du télogène (I) pour former le copolymère à blocs.

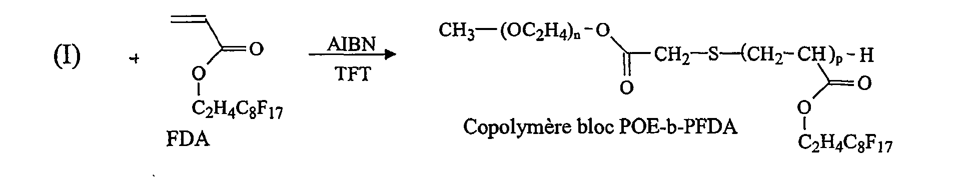

### 1.1. Synthèse du télogène macromoléculaire (I)

Dans un tricol de 1 litre, équipé d'un Dean Stark, d'un réfrigérant, et d'une olive aimantée, on incorpore 50,03 g (25 mmoles) d'éther monométhylique du poly(oxyde d'éthylène) (Aldrich, M̅ₙ = 2000 g/mole), et 705,7 g de benzène. On sèche le polymère par entraînement azéotropique, à reflux (100°C en consigne), pendant 5 heures. Ensuite, on ajoute 12,0243 g (127,9 mmoles) d'acide thioglycolique (Acros, 98%, M = 92,11 g/mole), et 127 mg (0,658 mmoles) d'acide para toluène sulfonique monohydraté (Aldrich, 98,5%). On poursuit l'entraînement azéotropique pendant 24 heures en soutirant régulièrement l'eau formée accumulée dans le Dean Stark.

En fin de réaction, le milieu est ramené à température ambiante, filtré sur fritté N°4 pour éliminer quelques insolubles, puis concentré à l'évaporateur rotatif à 40°C. On récupère 59,97 g d'un produit visqueux qui cristallise à température ambiante. On le dissout dans 31 g de chloroforme (solution très visqueuse). On précipite cette solution, sous agitation, dans 4 litres d'éther éthylique préalablement refroidi au congélateur. On filtre sur fritté N°4 et on lave la galette dans le fritté avec encore 1 litre d'éther éthylique à température ambiante. On récupère 55,01 g de poudre blanche qu'on sèche 2 heures sous vide (2.10⁻² mmHg) à 35-40°C. On obtient finalement 45,87 g de poudre blanche (rendement = 88%).

### 1.2. Synthèse d'un copolymère POE-b-PFDA

Le monomère acrylate fluoré 1,1,2,2-tétrahydro-perfluorodécyl acrylate (FDA) est distillé sous vide (P = 3.10⁻² mmHg, T_{eb} - 70-71°C). Le 2,2'-azobisisobutyronitrile (AIBN) (Fluka, 98%) est recristallisé dans le méthanol. Le solvant trifluorotoluène (Lancaster, 99%) (TFT) est distillé sous pression partielle (20 mm Hg). La polymérisation est effectuée sous argon, dans un réacteur Schlenk bicol de 100 mL, fermé avec 2 bouchons à jupe rabattable, sous agitation magnétique (olive aimantée).

Le monomère fluoré, placé dans un pilulier, est désoxygéné par bullage à l'argon (pendant 45 min.). L'agent de transfert et l'amorceur sont placés en solution dans le trifluorotoluène, dans le Schlenk (léger chauffage au pistolet chauffant pour solubiliser le télogène). La solution est désoxygénée par bullage à l'argon (45 min.). Le monomère est ajouté, sous balayage d'argon, dans le Schlenk à l'aide d'une seringue en verre. Le Schlenk est fermé, puis placé dans un bain d'huile à la température voulue (65°C ou 80°C). Le milieu initial, trouble après l'addition du monomère, devient limpide à chaud et sous agitation après environ 1 minute. Des prélèvements sont effectués à travers le septum avec une seringue en verre, l'avancement de la réaction est suivi par RMN ¹H, et la polymérisation est stoppée par ajout d'hydroquinone. Le produit final est précipité dans l'éther éthylique (large excès), séché sous vide à 30-35°C, puis conservé au frais.

Un premier copolymère, nommé P39, est synthétisé avec les conditions suivantes:
FDA: m = 10,0522 g
TFT: m = 40,0 g (distillé)
AIBN: m = 4,9 mg
Télogène (I): m = 3,3194 g
T = 65°C
t₀+ 21h50 : arrêt de la réaction

En raison de sa non solubilité dans les solvants usuels, le copolymère ne peut être analysé sans ambiguïté par chromatographie d'exclusion stérique. Des analyses effectuées par RMN ¹H (après dissolution dans un mélange TFT/fréon 113), et par analyse élémentaire, permettent la détermination du rapport molaire du composant CO₂-phile (FDA) sur le composant hydrophile (POE). Considérant que le produit réactionnel peut contenir des homopolymères POE, ce rapport est la limite inférieure du rapport moyen des composants CO₂-philes sur les composants hydrophiles du copolymère à blocs formé lors de la synthèse. Les analyses montrent que le produit de réaction est composé majoritairement d'un copolymère dibloc POE-b-PFDA constitué d'un bloc hydrophile POE de masse molaire moyenne en nombre (Mn) 2090 g/mol provenant du télogène (I) et d'un bloc CO₂-phile PFDA d'une masse molaire moyenne en nombre égale ou supérieure à 4869 g/mol. Le rapport des blocs CO₂-phile et hydrophile, calculé comme le rapport de la masse molaire Mn du bloc PFDA CO₂-phile sur la masse molaire Mn du bloc POE hydrophile est donc pour ce polymère P39 égal ou supérieur à 2,4.

### Exemple 2: Synthèse d'une série de copolymère à blocs POE-b-PFDA

Une première série de copolymères à blocs POE-b-PFDA, nommés P43, P44, P49, P50, P52, basée sur le télogène (I) dérivé du POE 2000 a été synthétisée de manière à obtenir une gamme de copolymères à blocs de rapports massiques CO₂-phile sur hydrophile croissants. La composition du copolymère est modulée en variant la masse du monomère FDA et la masse du télogène engagées dans la synthèse. Une seconde série de copolymères à blocs POE-b-PFDA obtenus par télomérisation radicalaire conventionnelle à partir d'un télogène dérivé d'un POE de masse molaire Mn 5 000 g/mol, par une voie de synthèse analogue à celle décrite dans l'exemple 1, a également été synthétisée. Ils sont dénommés P45 et P48. Le tableau 1 présente la valeur moyenne inférieure du rapport massique CO2-phile sur hydrophile de ces copolymères, comme décrit dans l'exemple 1.

**Tableau 1**

| Copolymère | Limite inférieure du rapport PFDA/POE |
|---|---|
| P43 | 6,4 |
| P44 | 7,2 |
| P49 | 37,8 |
| P50 | 28 |
| P52 | 18,9 |
| P45 | 1,2 |
| P48 | 10,2 |

### Exemple 3: Détermination de la solubilité des copolymères dans le CO₂ à pression supercritique

La solubilité des copolymères décrit dans les exemples 1 et 2 est évaluée à l'aide d'un appareil décrit en détail dans le brevet EP 0 784 506 B1. Cet appareil est constitué d'un réacteur d'une pression d'utilisation maximale de 30 MPa et d'un volume d'un litre, équipé d'un dispositif d'agitation rotatif à entraînement magnétique. Le réacteur est équipé d'une double enveloppe à circulation d'eau pour assurer la régulation de la température. Une masse connue de copolymère est introduite dans un filtre papier de porosité 2 µm préalablement pesé. Le filtre est alors replié et fixé solidement sur la partie supérieure de l'axe d'entraînement du dispositif d'agitation de manière à assurer un confinement mécanique du copolymère dans ce filtre. L'ensemble est placé dans le réacteur. La vitesse d'agitation est fixée à 460 tours par minutes. Du dioxyde de carbone est introduit dans le réacteur jusqu'à une pression de 20 MPa à une température de 45°C. Le copolymère est extrait dans ces conditions de température et pression durant une heure. La température du réacteur est alors réduite à environ 23°C en environ 30 minutes. Le réacteur est ensuite graduellement mis à pression atmosphérique par ouverture d'une vanne micrométrique permettant une circulation du dioxyde de carbone vers une ligne d'évent à pression atmosphérique. Après ouverture du réacteur, le filtre est récupéré et pesé de manière à calculer la masse de la fraction solubilisée dans le CO₂ à pression supercritique. Le tableau 2 regroupe les résultats des mesures de solubilité pour les copolymères décrits dans les exemples 1 et 2.

**Tableau 2**

| Fraction solubilisée à 45°C et 20 MPa. | | |
|---|---|---|
| Copolymère | Prise d'essai (mg) | Fraction solubilisée (%) |
| P39 | 502,72 | 66,3 |
| P43 | 971,28 | 85,3 |
| P44 | 998,61 | 90,4 |
| P52 | 982,92 | 94,1 |
| P50 | 996,38 | 76 |
| P49 | 974,44 | 92,4 |
| P45 | 988,06 | 69,2 |
| P48 | 976,54 | 72,6 |

Ce dispositif de mesure de la solubilité dans le CO₂ à pression supercritique est utilisé en routine par le demandeur. Considérant que, lors de la réduction de pouvoir solvant du fluide, une partie de l'agent de revêtement solubilisé se dépose sur les surfaces du filtre, une masse extraite de l'ordre de 90% est couramment observée pour des composés connus comme totalement solubles dans le fluide à pression supercritique. Les copolymères synthétisés dans les exemples 1 et 2 sont donc solubles dans le CO₂ à pression supercritique.

### Exemple 4: Courbe de point trouble d'un copolymère

Afin de quantifier la solubilité des copolymères à bloc POE-b-PFDA dans le CO₂ à pression supercritique, la courbe de point trouble de la fraction extraite du copolymère P44 tel qu'obtenu dans l'exemple 3 a été établie à l'aide d'une cellule optique à volume variable.

Le tableau 3 présente les pressions de point trouble à différentes températures et à une fraction massique constante de copolymère de 1,2% par rapport à la masse de CO₂. A une pression supérieure à la pression de point trouble, il est observé visuellement la solubilité totale de l'échantillon. A une pression inférieure à la pression de point trouble, un trouble est observé visuellement suite à la précipitation du copolymère. Ces résultats confirment la solubilité du copolymère dans le CO₂ à pression supercritique.

**Tableau 3**

| Température (°C) | Pression (MPa) |
|---|---|
| 65,2 | 23,23 |
| 59,9 | 22,1 |
| 55,2 | 20,6 |
| 49,8 | 19,1 |
| 45 | 17,6 |
| 40,2 | 15,99 |
| 35,3 | 14,3 |
| 30,4 | 12,65 |
| 25,7 | 10,74 |
| 20,5 | 8,92 |

### Exemple 5 : Dispersion de particules dans le CO₂ supercritique

### 5.1. Mode opératoire

Les propriétés de stabilisation de dispersion de particules hydrophiles solides sont évaluées indirectement dans une expérience d'encapsulation telle que décrite dans le brevet EP 0 784 506 B1.

Le copolymère à bloc CO₂-phile/hydrophile est donc ici utilisé en tant qu'agent de dispersion et en tant qu'agent de revêtement. Le dispositif expérimental est un appareil d'encapsulation à l'aide de CO₂ supercritique similaire à celui utilisé dans l'exemple 3, équipé d'un réacteur d'un volume interne de 125 mL et d'une pression maximale d'utilisation de 30 MPa. Le réacteur est équipé de fenêtres optiques permettant l'observation du milieu à pression supercritique. 50 mg de particules sphériques de silice non greffée d'un diamètre de 50 µm (Nucleodur 100-50, Macherey-Nagel) sont introduits dans le réacteur. 200 mg de copolymère P39, décrit dans l'exemple 1, sont également introduits. Le réacteur est équipé d'une double enveloppe permettant de réguler sa température. Le réacteur est fermé à l'aide d'un couvercle équipé d'un dispositif d'agitation de type ancre. L'agitation est réglée à 460 tours par minutes. Du dioxyde de carbone est introduit dans le réacteur jusqu'à obtenir une pression de 20 MPa à une température de 45°C. Dans ces conditions, le copolymère est solubilisé. On observe alors une dispersion stable et homogène de particules de silice dans le CO₂ supercritique.

Après une heure, la température du réacteur est graduellement diminuée à 20°C en environ 50 minutes de manière à provoquer le dépôt du copolymère à la surface des particules. La pression du réacteur est alors diminuée jusqu'à la pression atmosphérique en environ 60 minutes en permettant l'échappement du dioxyde de carbone de manière contrôlée par ouverture d'une vanne micrométrique. Après ouverture du réacteur, les particules sont récupérées.

### 5.2. Résultats

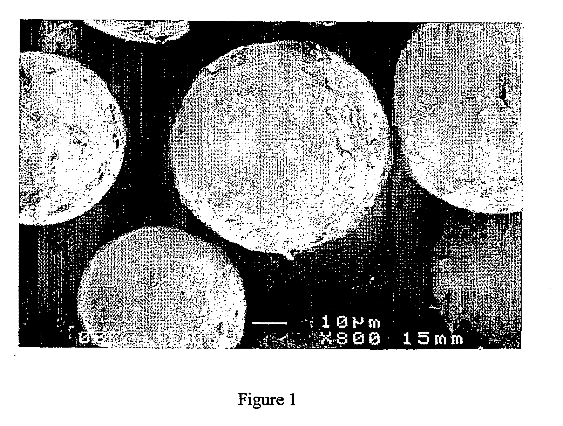

La figure 1 présente l'aspect des particules récupérées après avoir été soumises à ce procédé d'encapsulation.

Les particules initiales, qui sont restées individuelles, ont été recouvertes d'une couche homogène du copolymère sur la totalité de leur surface, témoignant de l'exposition de l'ensemble de leur surface à l'agent de revêtement. Aucune agglomération des particules n'est de plus notée. Lors de l'utilisation d'agents de revêtements hydrophobes comme par exemple des triglycérides, un phénomène d'agglomération des particules est couramment observé par le demandeur avec les mêmes conditions expérimentales.

Ce résultat démontre la capacité du copolymère à bloc POE-b-PFDA P39 à stabiliser une dispersion de particules hydrophiles dans le CO₂ à pression supercritique.

### Exemple 6: Formation d'émulsions en présence de CO₂ supercritique

### 6.1. Mode opératoire

L'aptitude des copolymères décrits dans les exemples 1 et 2 à stabiliser une émulsion est évaluée à l'aide d'une cellule d'observation des équilibres de phase à pression supercritique. Cette cellule est composée d'un cylindre en acier inox d'une pression maximale d'utilisation de 50 MPa, et d'un volume variant entre 50 et 64 mL. Le volume interne de la cellule est ajusté par l'intermédiaire d'un piston actionné par de l'azote sous pression. La température du milieu est régulée par circulation d'huile de silicone dans une double enveloppe. Le milieu peut être homogénéisé à l'aide d'un barreau aimanté mis en rotation par agitation magnétique. Le cylindre est équipé de deux fenêtres en saphir disposées en vis à vis. La cellule est éclairée par la fenêtre arrière. Le milieu est observé à travers la fenêtre située en avant du cylindre. 22,5 mL d'une solution aqueuse d'un colorant hydrophile rouge, l'amarante, sont introduits dans le cylindre haute pression. 200 mg du copolymère P44 sont également introduits dans la cellule. Le cylindre est alors fermé à l'aide couvercle équipé du piston de contre pression. Le volume est fixé à 50mL par l'intermédiaire du piston. La température du milieu est maintenue à 40°C. Le cylindre est purgé à l'aide d'un flux de dioxyde de carbone, puis du dioxyde de carbone est introduit jusqu'à une pression de 30 MPa en maintenant le volume à 50 mL. Lors de l'introduction du dioxyde de carbone, la poudre blanche de copolymère est progressivement solubilisée. Le milieu est alors agité et il se forme une phase dispersée rouge et opaque occupant la totalité du volume du cylindre.

Après 30 minutes, l'agitation est stoppée et un déphasage lent et progressif du système est observé en environ 5 minutes. Un système binaire est alors observé et ne subit pas d'évolution sur une durée de 2 heures. La partie inférieure de la cellule est rouge et opaque. La partie supérieure est rouge et opalescente. Il s'agit d'émulsion dans le CO₂, stables sans agitation pendant les deux heures de l'expérience.

Pour confirmer le rôle du copolymère dans la formation de cette émulsion, la pression est graduellement diminuée par remontée progressive du piston. Il se produit alors une déstabilisation de l'émulsion et on observe une phase supérieure devenant incolore et translucide et une phase inférieure contenant des agrégats blanc correspondant au copolymère non solubilisé en raison de la diminution du pouvoir solvant du dioxyde supercritique. Une augmentation de pression permet de solubiliser à nouveau le copolymère et de former à nouveau une émulsion eau dans CO₂

### 6.2. Résultats

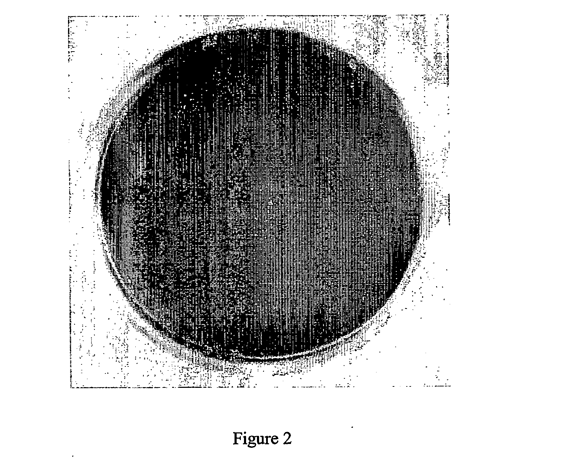

La figure 2 représente une photographie du milieu vu depuis la fenêtre avant du réacteur. Le copolymère a donc permis de disperser sous forme d'émulsion la solution aqueuse de colorant dans le dioxyde de carbone supercritique à une température de 40°C et une pression de 30 MPa. Une émulsion composée de gouttelettes de la solution aqueuse du colorant dans le CO₂ est formée.

### Exemple 7: Exemple comparatif sans utilisation de copolymères à blocs

### 7.1. Mode opératoire

La procédure expérimentale décrite dans l'exemple 6 est utilisée, mais seule la solution aqueuse d'amarante et le dioxyde de carbone sont introduits dans la cellule.

### 7.2. Résultats

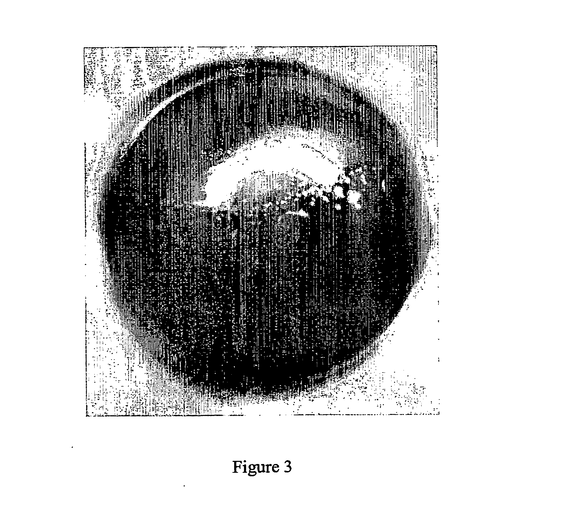

En l'absence de copolymère et après une heure d'agitation, deux phases distinctes sont observées dans la cellule sous agitation (figure 3). La phase dense est translucide et rouge, la phase supérieure est translucide et incolore. Il ne s'est donc ni formé d'émulsion ni produit de solubilisation du colorant hydrophile dans le dioxyde de carbone supercritique à une température de 40°C et une pression de 30 MPa.

Un comportement identique est observé avec un volume de solution aqueuse d'amarante de 7,5mL et du dioxyde de carbone liquide à une température de 20°C et une pression de 20 MPa.

Cet exemple comparatif confirme le rôle du copolymère à blocs POE-b-PFDA dans la formation d'émulsion eau dans CO₂ à pression supercritique.

### Exemple 8: Formation d'une émulsion dans le CO₂ liquide à pression supercritique

Une expérience similaire à celle de l'exemple 6 est menée, à l'exception de la température qui est fixée à 20°C et du volume de solution aqueuse fixé à 7,5 mL.

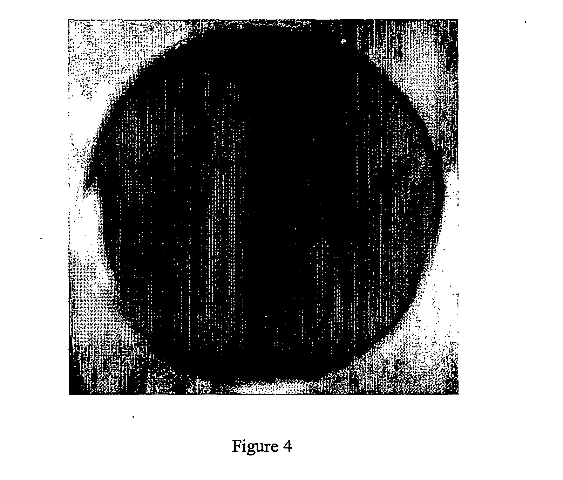

A cette température, et dans les conditions de pression utilisées, le dioxyde de carbone à pression supercritique se trouve à l'état liquide. A 20°C et 30 MPa, une émulsion d'eau dans le CO₂ liquide est formée après agitation comme présentée dans la figure 4.

Le copolymère à blocs POE-b-PFDA a donc permis de former une émulsion d'une solution aqueuse dans le CO₂ liquide à pression supercritique.

### Exemple 9: Dispersion de particules hydrophiles dans le CO2 supercritique

Les particules hydrophiles utilisées dans cet exemple sont des particules contenant des excipients typiquement utilisés pour formuler des lyophilisats contenant des protéines thérapeutiques. Les particules hydrophiles sont obtenues par lyophilisation de 100 mL d'une solution aqueuse contenant 8,39 g de glycine, 410 mg d'albumine sérique humaine, 2,4 g d'hydrogénophosphate de sodium dodécahydraté et 266 mg de dihydrogénophosphate de sodium dihydraté. Après lyophilisation, les particules sont broyées à l'aide d'un mortier en agate puis tamisées à l'aide d'un tamis de maille 25 µm. Les particules tamisées de taille inférieure à 25 µm sont récupérées et utilisées dans la suite.

L'aptitude des copolymères décrits dans les exemples 1 et 2 à stabiliser une dispersion de particules hydrophiles est évaluée à l'aide de la cellule d'observation des équilibres de phase décrite dans l'exemple 6. 20 mg de particules hydrophiles et 200 mg de copolymère POE-b-PFDA P44 décrit dans l'exemple 2 sont introduits dans le cylindre haute pression de la cellule d'observation. La température du cylindre est ajustée à 45°C. Le volume est fixé à 50 mL par l'intermédiaire du piston de contre-pression. Le milieu est agité à l'aide d'un barreau magnétique. Du dioxyde de carbone est introduit dans la cellule jusqu'à l'obtention d'une pression de 22 MPa. A cette pression, le copolymère est solubilisé et les particules sont en suspension dans le milieu. Aucune particule n'est observée sur les parois du cylindre ou des fenêtres en saphir. La totalité des particules hydrophiles est donc dispersée dans le milieu agité en présence de copolymère à bloc POE-b-PFDA P44. L'agitation est alors stoppée. Une sédimentation lente des particules est observée. Le temps de sédimentation, déterminé par observation du milieu, est de 10 minutes en présence du copolymère à bloc POE-b-PFDA P44.

### Exemple 10: Dispersion des particules hydrophiles dans le CO₂ liquide

Une expérience identique est réalisée à une température de 25°C et une pression de 11,5 MPa. Lorsque le milieu est agité, la totalité des particules de lyophilisat hydrophile est dispersée dans le CO₂ liquide et aucune particule n'est observée sur les parois. L'agitation est alors stoppée. Une sédimentation lente des particules est observée, avec un temps de sédimentation de l'ordre de 10 minutes.

### Exemple 11: Exemple comparatif sans utilisation de copolymère à blocs

### 11.1. Dans le CO₂ supercritique.

La procédure expérimentale décrite dans l'exemple 9 est suivie, mais seules les particules hydrophiles sont introduites dans la cellule avant introduction de CO₂, sans copolymère à blocs P44.

Le cylindre haute pression est thermostaté à 45°C. La pression est augmentée jusqu'à 22 MPa par introduction de CO₂. Le milieu est agité par l'intermédiaire d'un barreau aimanté. Il est observé que la plus grande partie des particules est présente sur les parois internes du cylindre haute pression et des fenêtres en saphir et n'est pas dispersée dans le fluide supercritique. Quelques rares fines particules sont toutefois dispersées dans le CO₂ supercritique. Dès arrêt de l'agitation, une sédimentation rapide de ces rares particules dispersées est observée. Le temps de sédimentation, déterminé en observant le milieu à travers la fenêtre en saphir, est dans ce cas d'environ 90 secondes.

### 11.2. Dans le CO₂ liquide.

Des résultats comparables sont obtenus en tentant de disperser ces particules dans le CO₂ liquide selon la procédure décrite dans l'exemple 10, à une température de 25°C à une pression de 20 MPa. La plus grande partie des particules est observée sur les parois de la cellule et les fenêtres en saphir. Quelques rares particules sont dispersées dans le CO₂ liquide si le milieu est agité. A l'arrêt de l'agitation, le temps de sédimentation est d'environ 2 minutes.

### 11.3. Conclusion.

En l'absence de copolymères à blocs, les particules hydrophiles ne sont donc pas efficacement dispersées dans le CO₂ supercritique ou liquide. Une agitation vigoureuse du milieu ne permet de disperser qu'une faible proportion de particules dans le CO₂ à pression supercritique, la plus grande partie des particules étant trouvée sur les parois du cylindre haute pression. Un arrêt de l'agitation provoque une sédimentation très rapide des rares particules hydrophiles dispersées par agitation.

Ces résultats, comparés à ceux obtenus dans les exemples 9 et 10, confirment qu'à 25°C ou 45°C, donc pour du CO₂ liquide ou supercritique, l'utilisation de copolymères à blocs POE-b-PFDA permet de disperser de manière efficace des particules hydrophiles dans le CO₂ à pression supercritique.

## Revendications

1. Procédé d'encapsulation d'un principe actif comprenant une étape de dispersion de substances hydrosolubles ou hydrophiles qui contiennent un principe actif choisi dans le groupe comprenant (i) les produits pharmaceutiques, notamment les analgésiques, les antipyrétiques, l'aspirine et ses dérivés, les antibiotiques, les anti-inflammatoires, les antiulcéreux, les antihypertenseurs, les neuroleptiques, les antidépresseurs, les oligonucléotides présentant une activité thérapeutique, les peptides présentant une activité thérapeutique et les protéines présentant une activité thérapeutique, (ii) les produits cosmétiques, notamment les autobronzants et les anti-UV, (iii) les produits alimentaires comme par exemple les vitamines, dans un fluide à pression supercritique par addition d'un surfactant, ledit surfactant étant un copolymère à blocs comprenant au moins un bloc CO₂-phile et au moins un bloc hydrophile non ionique.

2. Procédé selon la revendication 1, **caractérisé en ce que** le fluide à pression supercritique est du CO₂.

3. Procédé selon la revendication 1, **caractérisé en ce que** le fluide à pression supercritique est du CO₂ contenant un entraîneur dans une quantité inférieure à 5%.

4. Procédé selon la revendication 1, **caractérisé en ce que** le bloc CO₂-phile est choisi dans le groupe comprenant les polymères solubles dans le CO₂ à pression supercritique.

5. Procédé selon l'une quelconque des revendications 1 à 4, **caractérisé en ce que** les copolymères à blocs sont des copolymères solubles dans le CO₂ supercritique.

6. Procédé selon la revendication 5, **caractérisé en ce que** la solubilité minimale des copolymères à blocs est de 0,05% m/m, et de préférence de 0,2% m/m, à au moins une température définie et comprise entre 0°C et 100°C, de préférence définie et comprise entre 15°C et 60°C, et à au moins une pression définie et supérieure à la pression critique du CO₂, de préférence inférieure à 70 MPa, et encore plus préférentiellement inférieure à 30 MPa.

7. Procédé selon l'une quelconque des revendications 1 à 6 **caractérisé en ce que** la masse molaire moyenne en nombre du copolymère à blocs est choisie entre 1 000 et 200 000 g/mol, de préférence entre 4 000 et 50 000 g/mol.

8. Procédé selon la revendication 7, **caractérisé en ce que** la masse molaire moyenne en nombre du bloc hydrophile est comprise entre 500 et 20 000 g/mol, de préférence entre 1 000 et 10 000 g/mol.

9. Procédé selon l'une quelconque des revendications 1 à 8, **caractérisé en ce que** le rapport massique du bloc CO₂-phile sur le bloc hydrophile, est compris entre 1 et 50, de préférence entre 1 et 20.

10. Procédé selon l'une quelconque des revendications 1 à 9, **caractérisé en ce que** le bloc CO₂-phile du copolymère à blocs est choisi dans le groupe comprenant les polymères fluorés et les poly(siloxanes)

11. Procédé selon la revendication 10 **caractérisé en ce que** le polymère fluoré est choisi dans le group e comprenant 1 es poly(fluoroéthers) et les poly(fluoroalkyl acrylates) et les poly(fluoroalkyl méthacrylates).

12. Procédé selon la revendication 11 **caractérisé en ce que** les poly(fluoroalkyl acrylates) sont les poly(1,1-dihydroperfluorooctylacrylates) et les poly(1,1,2,2-tétrahydroperfluorodecylacrylates).

13. Procédé selon la revendication 1, **caractérisé en ce que** le bloc hydrophile non ionique est choisi parmi les polymères hydrophiles biocompatibles.

14. Procédé selon la revendication 13, **caractérisé en ce que** les polymères hydrophiles biocompatibles sont choisis dans le groupe comprenant les polysaccharides, les polymères cellulosiques hydrophiles, l'alcool polyvinylique, les polyols, les homo et copolymères d'oxyde d'éthylène.

15. Procédé selon la revendication 14 **caractérisé en ce que** le bloc hydrophile est un poly(oxyde d'éthylène).

16. Procédé selon l'une quelconque des revendications 1 à 15, **caractérisé en ce que** les copolymères à blocs sont composés d'un bloc poly(1,1,2,2-tétrahydro perfluorodécyl acrylates) et d'un bloc poly(oxyde d'éthylène) ou sont des copolymères à blocs du type POE-b-PFDA, ou sont choisis dans le groupe comprenant les copolymères PFDA-b-POE- PFDA et les copolymères triblocs POE-b-PFDA-b-POE.

17. Procédé selon les revendications 1 à 16 **caractérisé en ce que** les protéines ou les peptides thérapeutiques sont choisis dans le groupe comprenant la protéine correspondant à l'hormone parathyroïde, l'hormone de croissance, les interférons α, β, ou γ, l'érythropoiétine α ou β, (EPO), le facteur stimulateur de colonies de granulocytes (GCSF), le facteur stimulateur de colonies macrophages de granulocytes (GMSCF), le peptide vasoactif intestinal (VIP), l'hormone libératrice de thyrotopine (CRH), l'arginine vasopressine (AVP), l'angiotensine, l'insuline, la somatotropine, l'activateur de tissu plasminogène, les facteurs de coagulations VIII et IX, la glucosylcéramidase, la lénograstine, la molgramostine, la filgrastine, les interleukines, la dornase α, la PEG-L-asparaginase, la PEG-adenosine deaminase, l'hirudine, l'eptacog α, les facteurs de croissances nerveuses, l'hormone libératrice d'hormone lutéinisante (LHRH) , ses dérivés et ses analogues, la somatostatine et ses dérivés, la triptoréline, la bombésine, la calcitonine, le peptide libérateur de gastride, le facteur libérateur d'hormone de croissance et l'amyline.

18. Procédé selon l'une quelconque des revendications précédentes, **caractérisé en ce que** le copolymère à blocs comprend au moins un bloc CO₂-phile et au moins un bloc hydrophile non ionique biocompatible.

19. Procédé selon l'une quelconque des revendications précédentes, **caractérisé en ce que** les copolymères à blocs sont choisis dans le groupe constitué par les copolymères diblocs et les copolymères triblocs.

20. Procédé selon la revendication 19, **caractérisé en ce que** le copolymère tribloc répond soit à la formule (1)
hydrophile/CO₂-phile/hydrophile (1),
soit à la formule (2)
CO₂-phile/hydrophile/CO₂-phile (2),
dans lesquelles respectivement les groupes hydrophiles ou CO₂-philes peuvent être identiques ou différents.

21. Procédé selon l'une quelconque des revendications précédentes, **caractérisé en ce que** le bloc CO₂-phile est choisi dans le groupe comprenant des polymères fluorés et les poly(siloxanes).

22. Procédé selon la revendication 21, **caractérisé en ce que** le polymère fluoré est choisi dans le groupe comprenant les poly(fluoroéthers), les poly(fluoroalkyl méthacrylates) et les poly(fluoroalkyl acrylates).

23. Procédé selon la revendication 22, **caractérisé en ce que** les poly(fluoroalkylacrylates) sont des poly(1,1-dihydro-perfluorooctyl acrylates) et plus particulièrement des poly(1,1,2,2-tétrahydro-perfluorodécyl acrylates).

24. Procédé selon la revendication 18, **caractérisé en ce que** le bloc hydrophile non ionique biocompatible est choisi dans le groupe comprenant les polysaccharides, les polymères cellulosiques hydrophiles, l'alcool polyvinylique, les polyols, les homo- et copolymères d'oxyde d'éthylène.

25. Procédé selon la revendication 24, **caractérisé en ce que** le bloc hydrophile est un poly(oxyde d'éthylène).

26. Procédé selon la revendication 25, **caractérisés en ce qu'**ils sont composés d'un bloc poly(1,1,2,2-tétrahydro-perfluorodécyl acrylates) et d'un bloc poly(oxyde d'éthylène), ou sont des copolymères à blocs du type POE-b-PFDA, ou sont choisis dans le groupe comprenant les copolymères PFDA-b-POE-PFDA et les copolymères triblocs POE-b-PFDA-b-POE.

## Claims

1. Process for the encapsulation of an active principle, comprising a stage of dispersion of watersoluble or hydrophilic substances which comprise an active principle chosen from the group consisting of (i) pharmaceuticals, in particular analgesics, antipyretics, aspirin and its derivatives, antibiotics, anti-inflammatories, antiulceratives, antihypertensives, neuroleptics, antidepressants, oligonucleotides exhibiting a therapeutic activity, peptides exhibiting a therapeutic activity and proteins exhibiting a therapeutic activity, (ii) cosmetics, in particular self-tanning agents and UV stabilizers, and (iii) foodstuffs, such as, for example, vitamins, in a fluid at supercritical pressure by addition of a surfactant, the said surfactant being a block copolymer comprising at least one CO₂-philic block and at least one nonionic hydrophilic block.

2. Process according to Claim 1, **characterized in that** the fluid at supercritical pressure is CO₂.

3. Process according to Claim 1, **characterized in that** the fluid at supercritical pressure is CO₂ comprising an entrainer in an amount of less than 5%.

4. Process according to Claim 1, **characterized in that** the CO₂-philic block is chosen from the group consisting of polymers which are soluble in CO₂ at supercritical pressure.

5. Process according to any one of Claims 1 to 4, **characterized in that** the block copolymers are copolymers which are soluble in supercritical CO₂.

6. Process according to Claim 5, **characterized in that** the minimum solubility of the block copolymers is 0.05% w/w and preferably 0.2% w/w at at least one defined temperature which is between 0°C and 100°C, preferably at least one defined temperature which is between 15°C and 60°C, and at at least one defined pressure which is greater than the critical pressure of CO₂, preferably less than 70 MPa and more preferably still less than 30 MPa.

7. Process according to any one of Claims 1 to 6, **characterized in that** the number-average molar mass of the block copolymer is chosen between 1000 and 200 000 g/mol, preferably between 4000 and 50 000 g/mol.

8. Process according to Claim 7, **characterized in that** the number-average molar mass of the hydrophilic block is between 500 and 20 000 g/mol, preferably between 1000 and 10 000 g/mol.

9. Process according to any one of Claims 1 to 8, **characterized in that** the ratio by weight of the CO₂-philic block to the hydrophilic block is between 1 and 50, preferably between 1 and 20.

10. Process according to any one of Claims 1 to 9, **characterized in that** the CO₂-philic block of the block copolymer is chosen from the group consisting of fluoropolymers and poly(siloxane)s.

11. Process according to Claim 10, **characterized in that** the fluoropolymer is chosen from the group consisting of poly(fluoroether)s, poly(fluoroalkyl acrylate)s and poly(fluoroalkyl methacrylate)s.

12. Process according to Claim 11, **characterized in that** the poly(fluoroalkyl acrylate)s are poly(1,1-dihydroperfluorooctyl acrylate)s and poly(1,1,2,2-tetrahydroperfluorodecyl acrylate)s.

13. Process according to Claim 1, **characterized in that** the nonionic hydrophilic block is chosen from biocompatible hydrophilic polymers.

14. Process according to Claim 13, **characterized in that** the biocompatible hydrophilic polymers are chosen from the group consisting of polysaccharides, hydrophilic cellulose polymers, poly(vinyl alcohol), polyols, and ethylene oxide homo- and copolymers.

15. Process according to Claim 14, **characterized in that** the hydrophilic block is a poly(ethylene oxide).

16. Process according to any one of Claims 1 to 15, **characterized in that** the block copolymers are composed of a poly(1,1,2,2-tetrahydroperfluoro-decyl acrylate)s block and of a poly(ethylene oxide) block or are block copolymers of the PEO-b-PFDA type, or are chosen from the group consisting of PFDA-b-PEO-b-PFDA triblock copolymers and PEO-b-PFDA-b-PEO triblock copolymers.

17. Process according to Claims 7 to 16, **characterized in that** the therapeutic proteins or peptides are chosen from the group consisting of the protein corresponding to parathyroid hormone, growth hormone, α-, p- or γ-interferons, α- or β-erythropoietin (EPO), granulocyte colony-stimulating factor (GCSF), granulocyte-macrophage colony-stimulating factor (GMCSF), vasoactive intestinal peptide (VIP), thyrotropin-releasing hormone (TRH), arginine vasopressin (AVP), angiotensin, insulin, somatotropin, tissue plasminogen activator, clotting factors VIII and IX, glucosylceramidase, lenograstim, molgramostim, filgrastim, interleukins, dornase alfa, PEG-L-asparaginase, PEG-adenosine deaminase, hirudin, eptacog alfa, nerve growth factors, luteinizing hormone-releasing hormone (LHRH), its derivatives and its analogues, somatostatin and its derivatives, triptorelin, bombesin, calcitonin, gastrin-releasing peptide, growth hormone-releasing factor and amylin.

18. Process according to any one of the preceding claims, **characterized in that** the block copolymer comprises at least one CO₂-philic block and at least one biocompatible nonionic hydrophilic block.

19. Process according to any one of the preceding claims, **characterized in that** the block copolymers are chosen from the group consisting of diblock copolymers and triblock copolymers.

20. Process according to Claim 19, **characterized in that** the triblock copolymer corresponds either to the formula (1)
hydrophiliC/CO₂-philic/hydrophilic (1),
or to the formula (2)
CO₂-philic/hydrophiliC/CO₂-philic (2),
in which, respectively, the hydrophilic or CO₂-philic groups can be identical or different.

21. Process according to any one of the preceding claims, **characterized in that** the CO₂-philic block is chosen from the group consisting of fluoropolymers and poly(siloxane)s.

22. Process according to Claim 21, **characterized in that** the fluoropolymer is chosen from the group consisting of poly(fluoroether)s, poly(fluoroalkyl methacrylate)s and poly(fluoroalkyl acrylate)s.

23. Process according to Claim 22, **characterized in that** the poly(fluoroalkyl acrylate)s are poly(1,1-dihydroperfluorooctyl acrylate)s and more particularly poly(1,1,2,2-tetrahydroperfluorodecyl acrylate)s.

24. Process according to Claim 18, **characterized in that** the biocompatible nonionic hydrophilic block is chosen from the group consisting of polysaccharides, hydrophilic cellulose polymers, poly(vinyl alcohol), polyols, and ethylene oxide homo- and copolymers.

25. Process according to Claim 24, **characterized in that** the hydrophilic block is a poly(ethylene oxide).

26. Process according to Claim 25, **characterized in that** the block copolymers are composed of a poly(1,1,2,2-tetrahydroperfluorodecyl acrylate)s block and of a poly(ethylene oxide) block or are block copolymers of the PEO-b-PFDA type, or are chosen from the group consisting of PFDA-b-PEO-b-PFDA triblock copolymers and PEO-b-PFDA-b-PEO triblock copolymers.

## Patentansprüche

1. Verfahren zur Verkapselung eines Wirkstoffs, umfassend einen Schritt einer Dispergierung von wasserlöslichen oder hydrophilen Substanzen, die einen Wirkstoff enthalten, ausgewählt in der Gruppe, umfassend (i) die pharmazeutischen Produkte, insbesondere die Analgetika, die Antipyretika, Aspirin und dessen Derivate, die Antibiotika, die entzündungshemmenden Mittel, die Ulkustherapeutika, die blutdrucksenkenden Mittel, die Neuroleptika, die Antidepressiva, die Oligonukleotide, welche eine therapeutische Aktivität aufweisen, die Peptide, welche eine therapeutische Aktivität aufweisen, und die Proteine, welche eine therapeutische Aktivität aufweisen, (ii) die kosmetischen Produkte, insbesondere die Selbstbräuner und die UV-Schutzmittel, (iii) die Nahrungsmittelerzeugnisse, wie beispielsweise die Vitamine, in einem Fluid bei überkritischem Druck durch Zugeben eines grenzflächenaktiven Mittels, wobei das grenzflächenaktive Mittel ein Blockcopolymer ist, umfassend wenigstens einen CO₂-philen Block und wenigstens einen hydrophilen, nicht-ionischen Block.

2. Verfahren nach Anspruch 1, **dadurch gekennzeichnet, dass** das Fluid bei überkritischem Druck CO₂ ist.

3. Verfahren nach Anspruch 1, **dadurch gekennzeichnet, dass** das Fluid bei überkritischem Druck CO₂ ist, welches einen Träger in einer Menge unter 5% enthält.

4. Verfahren nach Anspruch 1, **dadurch gekennzeichnet, dass** der CO₂-phile Block in der Gruppe, welche die in CO₂ bei überkritischem Druck löslichen Polymere umfasst, ausgewählt wird.

5. Verfahren nach einem der Ansprüche 1 bis 4, **dadurch gekennzeichnet, dass** die Blockcopolymere in überkritischem CO₂ lösliche Copolymere sind.

6. Verfahren nach Anspruch 5, **dadurch gekennzeichnet, dass** die minimale Löslichkeit der Blockcopolymere 0,05% (Masse/Masse) und vorzugsweise 0,2% (Masse/Masse) bei zumindest einer definierten und zwischen 0°C und 100°C eingeschlossen, vorzugsweise definierten und zwischen 15°C und 60°C eingeschlossen liegenden Temperatur und bei zumindest einem definierten und über dem kritischen Druck des CO₂, vorzugsweise unter 70 MPa und noch mehr bevorzugt unter 30 MPa liegenden Druck ist.

7. Verfahren nach einem der Ansprüche 1 bis 6, **dadurch gekennzeichnet, dass** das Molekulargewicht-Zahlenmittel des Blockcopolymers zwischen 1 000 und 200 000 g/mol eingeschlossen, vorzugsweise zwischen 4 000 und 50 000 g/mol eingeschlossen ausgewählt wird.

8. Verfahren nach Anspruch 7, **dadurch gekennzeichnet, dass** das Molekulargewicht-Zahlenmittel des hydrophilen Blocks zwischen 500 und 20 000 g/mol eingeschlossen, vorzugsweise zwischen 1 000 und 10 000 g/mol eingeschlossen liegt.

9. Verfahren nach einem der Ansprüche 1 bis 8, **dadurch gekennzeichnet, dass** das Massenverhältnis des CO₂-philen Blocks zu dem hydrophilen Block zwischen 1 und 50 eingeschlossen, vorzugsweise zwischen 1 und 20 eingeschlossen liegt.

10. Verfahren nach einem der Ansprüche 1 bis 9, **dadurch gekennzeichnet, dass** der CO₂-phile Block des Blockcopolymers in der Gruppe, umfassend die fluorierten Polymere und die Poly(siloxane), ausgewählt wird.

11. Verfahren nach Anspruch 10, **dadurch gekennzeichnet, dass** das fluorierte Polymer in der Gruppe, umfassend die Poly(fluorether) und die Poly(fluoralkylacrylate) und die Poly-(fluoralkylmethacrylate), ausgewählt wird.

12. Verfahren nach Anspruch 11, **dadurch gekennzeichnet, dass** die Poly(fluoralkylacrylate) die Poly(1,1-dihydroperfluoroctylacrylate) und die Poly(1,1,2,2-tetrahydroperfluordecylacrylate) sind.

13. Verfahren nach Anspruch 1, **dadurch gekennzeichnet, dass** der hydrophile, nicht-ionische Block unter den biologisch verträglichen hydrophilen Polymeren ausgewählt wird.

14. Verfahren nach Anspruch 13, **dadurch gekennzeichnet, dass** die biologisch verträglichen hydrophilen Polymere in der Gruppe, umfassend die Polysaccharide, die hydrophilen Cellulose-Polymere, Polyvinylalkohol, die Polyole, die Homo- und Copolymere von Ethylenoxid, ausgewählt werden.

15. Verfahren nach Anspruch 14, **dadurch gekennzeichnet, dass** der hydrophile Block ein Poly(ethylenoxid) ist.

16. Verfahren nach einem der Ansprüche 1 bis 15, **dadurch gekennzeichnet, dass** die Blockcopolymere aus einem Poly(1,1,2,2-tetrahydroperfluordecylacrylate)-Block und einem Poly(ethylenoxid)-Block aufgebaut sind oder Blockcopolymere vom Typ POE-b-PFDA sind oder in der Gruppe, umfassend die PFDA-b-POE-PFDA-Copolymere und die POE-b-PFDA-b-POE-Triblockcopolymere, ausgewählt werden.

17. Verfahren nach den Ansprüchen 1 bis 16, **dadurch gekennzeichnet, dass** die therapeutischen Proteine oder Peptide ausgewählt werden in der Gruppe, umfassend das Protein, welches dem Parathormon, dem Wachstumshormon, den Interferonen α, β oder γ, Erythropoietin α oder β (EPO), dem Granulozyten-koloniestimulierenden Faktor (GCSF), dem Granulozyten-Makrophagen-koloniestimulierenden Faktor (GMSCF), dem vasoaktiven intestinalen Peptid (VIP), dem Thyreotropin-releasing Hormon (TRH), Arginin-Vasopressin (AVP), Angiotensin, Insulin, Somatotropin, dem Plasminogengewebeaktivator, den Gerinnungsfaktoren VIII und IX, der Glucosylceramidase, Lenogastrin, Molgramostin, Filgrastin, den Interleukinen, der Dornase α, der PEG-L-Asparaginase, der PEG-Adenosindesaminase, Hirudin, Eptacog α, den Nervenwachstumsfaktoren, dem LH-releasing-Hormon (LH-RH), dessen Derivaten und dessen Analogen, Somatostatin und dessen Derivaten, Triptorelin, Bombesin, Calcitonin, dem Gastrid freisetzenden Peptid, dem Wachstumshormon freisetzenden Faktor und Amylin entspricht.

18. Verfahren nach einem der vorangegangenen Ansprüche, **dadurch gekennzeichnet, dass** das Blockcopolymer wenigstens einen CO₂-philen Block und wenigstens einen biologisch verträglichen hydrophilen, nicht-ionischen Block umfasst.

19. Verfahren nach einem der vorangegangenen Ansprüche, **dadurch gekennzeichnet, dass** die Blockcopolymere in der Gruppe, die aus den Diblockcopolymeren und den Triblockcopolymeren gebildet wird, ausgewählt werden.

20. Verfahren nach Anspruch 19, **dadurch gekennzeichnet, dass** das Triblockcopolymer entweder der Formel (1)
hydrophil/CO₂-phil/hydrophil (1)
oder der Formel (2)
CO₂-phil/hydrophil/CO₂-phil (2),
in welchen jeweils die hydrophilen bzw. CO₂-philen Gruppen identisch oder verschieden sein können, entspricht.

21. Verfahren nach einem der vorangegangenen Ansprüche, **dadurch gekennzeichnet, dass** der CO₂-phile Block in der Gruppe, welche die fluorierten Polymere und die Poly(siloxane) umfasst, ausgewählt wird.

22. Verfahren nach Anspruch 21, **dadurch gekennzeichnet, dass** das fluorierte Polymer in der Gruppe, umfassend die Poly(fluorether), die Poly(fluoralkylmethacrylate) und die Poly-(fluoralkylacrylate), ausgewählt wird.

23. Verfahren nach Anspruch 22, **dadurch gekennzeichnet, dass** die Poly(fluoralkylacrylate) Poly(1,1-dihydroperfluoroctylacrylate) und insbesondere Poly(1,1,2,2-tetrahydroperfluordecylacrylate) sind.

24. Verfahren nach Anspruch 18, **dadurch gekennzeichnet, dass** der biologisch verträgliche hydrophile, nicht-ionische Block in der Gruppe, umfassend die Polysaccharide, die hydrophilen Cellulose-Polymere, Polyvinylalkohol, die Polyole, die Homo- und Copolymere von Ethylenoxid, ausgewählt wird.

25. Verfahren nach Anspruch 24, **dadurch gekennzeichnet, dass** der hydrophile Block ein Poly(ethylenoxid) ist.

26. Verfahren nach Anspruch 25, **dadurch gekennzeichnet, dass** sie aus einem Poly(1,1,2,2-tetrahydroperfluordecylacrylate)-Block und einem Poly(ethylenoxid)-Block aufgebaut sind oder Blockcopolymere vom Typ POE-b-PFDA sind oder in der Gruppe, umfassend die PFDA-b-POE-PFDA-Copolymere und die POE-b-PFDA-b-POE-Triblockcopolymere, ausgewählt werden.
